# EUROPEAN PATENT APPLICATION

(11) **EP 1 886 989 A1**
(43) Date of publication of application: **13.02.2008**
(21) Application number: 06746906.4
(22) Date of filing: 26.05.2006
(51) Int. Cl.: C07C 69/736, G03F 7/039, H01L 21/027

(54) **CALIXRESORCINARENE COMPOUND, PHOTORESIST BASE COMPRISING THE SAME, AND COMPOSITION THEREOF**

(30) Priority: 01.06.2005 JP 2005161373
(71) Applicant: Watanabe, Takeo, Ako-gun, Hyogo 6781205 (JP); Kinoshita, Hiroo, Ako-gun, Hyogo 6781205 (JP); IDEMITSU KOSAN CO., LTD., Tokyo 100-8321 (JP)
(72) Inventor: OWADA, Takanori, 2990293 (JP); ISHII, Hirotoshi, 2990293 (JP); WATANABE, Takeo, Ako-gun, Hyogo 6781205 (JP); KINOSHITA, Hiroo, Ako-gun, Hyogo 6781205 (JP)
(74) Representative: Gille Hrabal Struck Neidlein Prop Roos
(86) International application number: PCT/JP2006/310577
(87) International publication number: WO 2006/129574

(57) **Abstract**

A calixresorcinarene compound represented by the following formula (1): 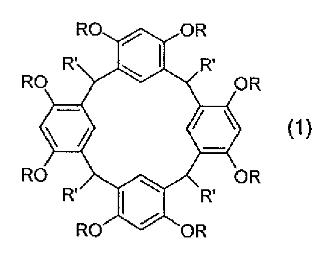 wherein eight Rs are n (n is an integer of 1 to 7) substituents that are one or more types of substituents selected from groups represented by the following formula (2), and m (m is an integer shown by 8-n) hydrogen atoms; and R's, which may be the same or different, are each a straight-chain aliphatic hydrocarbon group having 2 to 12 carbon atoms, a branched aliphatic hydrocarbon group having 3 to 12 carbon atoms, a phenyl group, a p-phenylphenyl group, a p-tert-butylphenyl group, and an aromatic group represented by the following formula (3), or a substituent formed by combining two or more of these substituents: 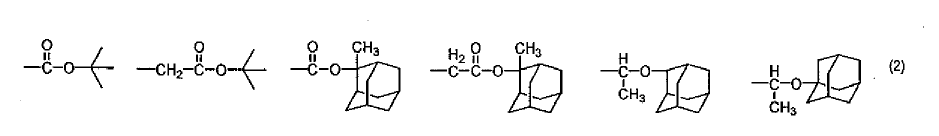 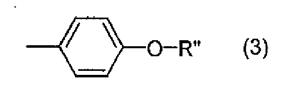 wherein R" is a hydrogen atom or a substituent selected from the substituents represented by the formula (2).

## Description

### TECHNICAL FIELD

The invention relates to a photoresist base material used in the fields of electricity and electronics such as a semiconductor, the optical field, and the like, in particular, to a photoresist base material for ultra-microfabrication.

### BACKGROUND

Lithography using extreme ultraviolet radiation (EUV) or an electron beam is useful as a high productivity and high resolution microfabrication method in the manufacture of semiconductors and the like. Development of a high sensitivity and high resolution photoresist for lithography is desired. Improvement of sensitivity is essential for a photoresist used in the lithography from a viewpoint of productivity of desired detailed patterns, high resolution, and the like.

As a photoresist used for ultra-microfabrication using extreme ultraviolet radiation, a chemically-amplified polyhydroxystyrene-based photoresist used for a known ultra-microfabrication using a KrF laser can be given, for example. This resist is known to be usable for microfabrication to a degree of about 50 nm. Formation of patterns finer than 50 nm is the greatest advantage of the ultra-microfabrication using extreme ultraviolet radiation. If such detailed patterns are produced using this resist, the line edge roughness, which is most important, cannot be lowered, though high sensitivity and reduced resist outgas can be realized to some extent. The resist thus cannot necessarily sufficiently derive excellent performance inherent to extreme ultraviolet radiation. Therefore, development of a photoresist exhibiting higher performance has been demanded.

In order to respond to such a demand, a method involving use of a chemically amplified positive-tone photoresist containing a higher concentration of a photoacid generator as compared with other resist compounds has been proposed (see Patent Document 1, for example). This Patent Document discloses a photoresist comprising a hydroxystyrene/styrene/t-butyl acrylate terpolymer base material, a photoacid generator of di(t-butylphenyl)iodonium ortho-trifluoromethylsulfonate, the amount of which is at least 5 wt% of the total solid content, lactate of tetrabutylammonium hydroxide, and ethyl lactate in examples. However, no specific results such as a line width obtained by using extreme ultraviolet radiation are described. Regarding the results attainable by using extreme ultraviolet radiation, it appears that microfabrication to the extent of 100 nm, shown in an example in which electron beams were used, is a limit in terms of line edge roughness. The main cause of this limitation is considered to be influences on the width and surface roughness of lines produced of a large three-dimensional morphology of an agglomerate of high-molecular-weight compounds or the molecule of an individual high-molecular-weight compound.

Although the inventors have already proposed a calixresorcinarene compound as a photoresist material with high sensitivity and high resolution (see Patent Document 2), there is a demand for a novel low-molecular-weight organic compound which is amorphous at room temperature. Further, improvement of various properties, including etching resistance which is a problem in the production of a semiconductor, is also desired.
Patent Document 1: JP-A-2002-055457
Patent Document 2: JP-A-2004-191913

An object of the invention is to provide an excellent photoresist base material and a composition thereof which have features such as high sensitivity, high resolution, and high microfabrication capability.

### SUMMARY OF THE INVENTION

The inventors have found that the problem involved in microfabrication using a conventional photoresist is caused by reactivity due to a three-dimensional molecular morphology or a molecular structure of a high-molecular-weight compound conventionally used as the photoresist base material, or the structure of protective groups in its molecular structure. The inventors have found a calixresorcinarene compound which rarely encounters these problems, leading to completion of the invention.
According to the invention, the following calixresorcinarene compound and the like is provided.
1. A calixresorcinarene compound represented by the following formula (1): 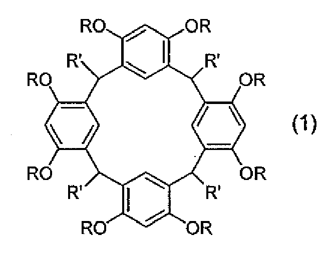 wherein eight Rs are n (n is an integer of 1 to 7) substituents that are one or more types of substituents selected from groups represented by the following formula (2), and m (m is an integer shown by 8-n) hydrogen atoms; and R's, which may be the same or different, are each a straight-chain aliphatic hydrocarbon group having 2 to 12 carbon atoms, a branched aliphatic hydrocarbon group having 3 to 12 carbon atoms, a phenyl group, a p-phenylphenyl group, a p-tert-butylphenyl group, and an aromatic group represented by the following formula (3), or a substituent formed by combining two or more of these substituents: 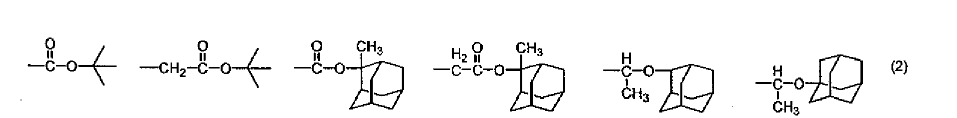 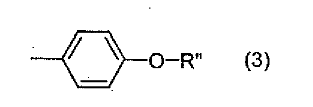 wherein R" is a hydrogen atom or a substituent selected from the substituents represented by the formula (2).
2. The calixresorcinarene compound according to 1, wherein R' is ethyl, n-propyl, iso-propyl, n-butyl, iso-butyl, tert-butyl, n-pentyl, n-hexyl, n-heptyl, n-octyl, n-nonyl, n-decyl, n-undecyl or n-dodecyl.
3. The calixresorcinarene compound according to 1, wherein R' is phenyl, p-phenylphenyl or p-tert-butylphenyl and a calixresorcinarene compound represented by the following formula (4): 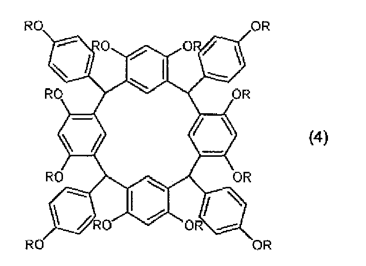 wherein twelve Rs are n' (n' is an integer of 1 to 11) substituents that are one or more types of substituents selected from groups represented by the following formula (2), and m' (m' is an integer represented by 12-n') hydrogen atoms. 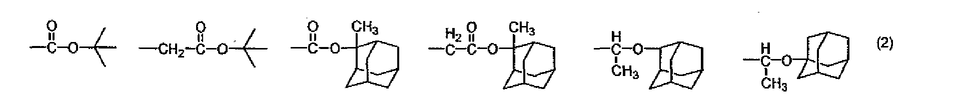
4. A photoresist base material comprising the calixresorcinarene compound according to any one of 1 to 3.
5. A photoresist composition comprising the photoresist base material according to 4 and a solvent.
6. A microfabrication method by lithography which uses the photoresist composition according to 5.
7. A microfabrication method by extreme ultraviolet lithography which uses the photoresist composition according to 5.
8. A semiconductor device which is formed by the microfabrication method according to 6 or 7.

The invention provides an excellent photoresist base material and a composition thereof which have features such as high sensitivity, high resolution, and high photolithographic processibility.

### BRIEF DESCRIPTION OF THE DRAWINGS

FIG. 1 is a ¹H-NMR spectrum chart of calixresorcinarene compound obtained in Synthesis Example 1.
FIG. 2 is a ¹H-NMR spectrum chart of calixresorcinarene compound obtained in Synthesis Example 2.
FIG. 3 is a ¹H-NMR spectrum chart of a calixresorcinarene compound obtained in Synthesis Example 3.
FIG. 4 is a ¹H-NMR spectrum chart of a calixresorcinarene compound obtained in Synthesis Example 4.
FIG. 5 is a ¹H-NMR spectrum chart of a calixresorcinarene compound obtained in Synthesis Example 5.
FIG. 6 is a ¹H-NMR spectrum chart of a photoresist base material obtained in Example 1.
FIG. 7 is a ¹H-NMR spectrum chart of a photoresist base material obtained in Example 2.
FIG. 8 is a ¹H-NMR spectrum chart of a photoresist base material obtained in Example 3.
FIG. 9 is a ¹H-NMR spectrum chart of a photoresist base material obtained in Example 4.
FIG. 10 is a ¹H-NMR spectrum chart of a photoresist base material obtained in Comparative Example 1.
FIG. 11 is a ¹H-NMR spectrum chart of polyhydroxystyrene used in Comparative Example 2.
FIG. 12 is a ¹H-NMR spectrum chart of a photoresist base material obtained in Comparative Example 2.

### BEST MODE FOR CARRYING OUT THE INVENTION

The calixresorcinarene compound of the invention is represented by the following formula (1): 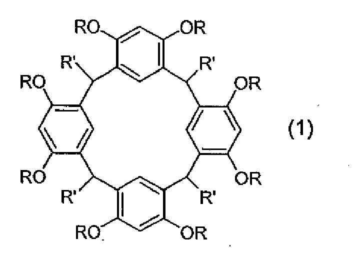 wherein eight Rs are n (n is an integer of 1 to 7) substituents that are one or more types of substituents selected from groups represented by the following formula (2), and m (m is an integer shown by 8-n) hydrogen atoms; and R's, which may be the same or different, are independently a straight-chain aliphatic hydrocarbon group having 2 to 12 carbon atoms, a branched aliphatic hydrocarbon group having 3 to 12 carbon atoms, a phenyl group, a p-phenylpheny group, a p-tert-butylphenyl group, and an aromatic group represented by the following formula (3), or a substituent formed by combining two or more of these substituents.

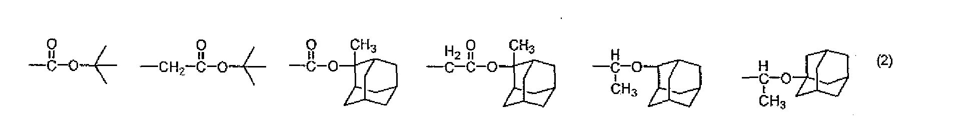

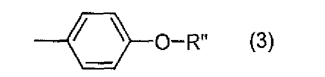

wherein R" is a hydrogen atom or a substituent selected from the substituents represented by the formula (2).

R' is preferably ethyl, n-propyl, iso-propyl, n-butyl, iso-butyl, tert-butyl, n-pentyl, n-hexyl, n-heptyl, n-octyl, n-nonyl, n-decyl, n-undecyl, n-dodecyl, phenyl, p-phenylphenyl or p-tert-butylphenyl.

In view of etching resistance, R' is more preferably n-propyl, n-butyl, iso-butyl, tert-butyl, n-pentyl, n-hexyl, n-heptyl, n-octyl, n-nonyl, n-decyl, n-undecyl, n-dodecyl, phenyl, p-phenylphenyl or p-tert-butylphenyl.

Since remarkable improvement is obtained in sensitivity, in addition to improvement in etching resistance, it is further preferred that R' be an aromatic group. Specific examples include a calixresorcinarene compound represented by formula (1) wherein R' is phenyl, p-phenylphenyl, and p-tert-butylphenyl, and a calixresorcinarene compound represented by the following formula (4) : 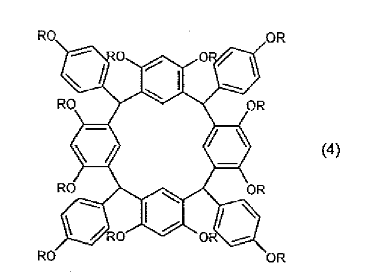 wherein twelve Rs are n' (n' is an integer of 1 to 11) substituents that are one or more types of substituents selected from groups represented by the following formula (2), and m' (m' is an integer represented by 12-n') hydrogen atoms. The substituent represented by the formula (2) is the same as those exemplified above.

The calixresorcinarene compound represented by the following formulas (1) and (4) is useful as a photoresist base material, particularly a photoresist base material used in ultra-microfabrication by lithography using extreme ultraviolet radiation, an electron beam or the like.
The photoresist base material of the invention contributes to a significant reduction of a resist outgas, as well as low line edge roughness in resolution. Since the Rs in the formulas (1) and (4) are directly or indirectly highly reactive to EUV and electron beam, the photoresist base material of the invention is not only excellent in sensitivity but also in etching resistance. Therefore, the photoresist base material of the invention can be suitably used as a photoresist base material for ultra-microfabrication.

The photoresist base material of the invention generates a decreased amount of resist outgas. The ring structure of the calixresorcinarene compound is considered to be the reason therefor, as explained below.
EUV is light which usually has an extremely short wavelength of around 13.5 nm. Therefore, every substance which is irradiated with EUV absorbs the EUV excited the inner core electrons, and bond cleavage occurs due to the inner core excitation at the time of EUV irradiation. It is difficult to completely suppress the bond cleavage in molecules of a photoresist base material in EUV lithography. A regist outgas is formed from low-molecular-weight compounds produced by the bond cleavage. Thus reducing the amount of the resist outgas is also considered to be difficult. The calixresorcinarene compound of the invention has a ring structure as the main chain structure, not a conventional straight chain, and therefore, two or more points must be cleaved to release a low-molecular-weight compound which constitutes a resist outgas. It is assumed that generation of a resist outgas from the ring structure is less likely to occur as compared with the conventional compound having a straight main chain structure. In the invention, the same advantageous effect is obtained when irradiation is performed by an electron beam. Similar effects can be confirmed as for suppression of resist outgas generation which is caused by bond cleavage which proceeds secondarily due to the effect of a chromophore such as a photoacid generator.

The compound of the invention is amorphous under the conditions in which a photoresist base material is used, usually at room temperature. Thus a photoresist composition using the compound as a base material exhibits excellent applicability and capability of producing a photoresist film with excellent strength.
Further, the compound of the invention has an average diameter of the molecule of less than a desired pattern size, specifically, less than the value of line edge roughness required for the size of 100 nm or less, and particularly 50 nm or less. For this reason, if the compound is used as a base material, the compound can suppress the line roughness value to 2 nm, preferably 1 nm or less (3σ), when used for processing in a range of 20 to 50 nm, featuring the ultra-microfabrication using extreme ultraviolet radiation or an electron beam.

When used as a photoresist base material, the compound may be used either singly or in combination of two or more insofar as the advantageous effects of the invention are not impaired.

When the calixresorcinarene compound of the invention is used as a photoresist base material, it is preferable to remove basic impurities (e.g. ammonia, alkaline metal ions such as Li, Na, and K, and alkaline earth metal ions such as Ca and Ba) by purification. In this case, it is preferred that the amount of basic impurities contained in the base material be reduced to 1/10 or less of the amount before purification.
Specifically, the amount of the impurities is preferably 10 ppm or less, more preferably 2 ppm or less.
By adjusting the content of the basic impurities to 10 ppm or less, sensitivity to extreme ultraviolet radiation or electron beam of the photoresist base material formed of this compound is remarkably improved. As a result, extremely minute patterns can be preferably formed by lithography from the photoresist composition.

The method for purification includes washing with an aqueous acidic solution and/or treatment with an ion exchange resin and/or re-precipitation with ultrapure water. As for the type of the aqueous acid solution and the ion exchange resin, the most suitable one can be appropriately selected depending on the amount or type of the basic impurities to be removed or the type of the base material to be treated. For example, treatment with an ion exchange resin or re-precipitation treatment with ultrapure water is conducted after washing with an aqueous acetic acid solution as the aqueous acidic solution.

The photoresist base material of the invention can be used as one component of the photoresist composition.
The photoresist composition of the invention comprises the photoresist base material of the invention and a solvent for dissolving the photoresist base material to form a liquid composition. The photoresist composition is required to be liquid so that it can be uniformly applied to a substrate and the like to be processed by ultra-microfabrication by a technique such as spin coating, dip coating, and painting.

As the solvent, any solvent which is commonly used as the solvent for a photoresist can be used. Examples include glycols such as 2-methoxyethyl ether, ethylene glycol monomethyl ether, propylene glycol monomethyl ether, and propylene glycol methyl ether acetate, lactates such as ethyl lactate and methyl lactate, propionates such as methyl propionate and ethyl propionate, cellosolve esters such as methyl cellosolve acetate, aromatic hydrocarbons such as toluene and xylene, ketones such as methyl ethyl ketone, cyclohexanone, and 2-heptanone. These solvents may be used either singly or in mixture. A preferred solvent can be determined according to the solubility for a solvent of an organic compound which is used as a base material, film-forming property, or the like.

The amount of other components than the solvent in the composition, i.e., the amount of photoresist solid matters, is preferably an amount suitable for forming a photoresist layer with a desired film thickness. Specifically, the amount is usually 0.1 to 50 wt% of the photoresist composition. The amount can be determined according to the type of the base material or solvent used, or a desired thickness or the like of a photoresist layer.

In the invention, when the molecule of a photoresist base material contains a chromophore active to EUV and/or an electron beam, the photoresist base material can exhibit performance as a photoresist by itself. A photoresist composition containing the base material need not contain a specified additive to the photoresist composition of the invention. However, if promotion of the performance as a photoresist is required, a photoacid generator (PAG) or the like is generally added as a chromophore, when necessary.

If necessary, in addition to a PAG, as a quenching agent to inhibit an overreaction of a PAG, an organic base, an anti-light splitting agent, a plasticizer, a speed promoter, a photosensitizer, a sensitizer, an acid growth functional material, an etching resistance reinforcing agent, and the like may be added. These additives may be used as a mixture of two or more components with the same or different functions, or as a mixture of precursors of these components. The composition of these components cannot be determined unconditionally since it depends on the type of the components used. In general, the similar composition as in a conventional photoresist can be applied.

As the PAG, in addition to known compounds of which the structures are exemplified below, other compounds having the same effect can be generally used. A preferable PAG and the amount thereof can be appropriately selected according to the type of calixresorcinarene compound as a base material, a desired shape and size of minute patterns, and the like. 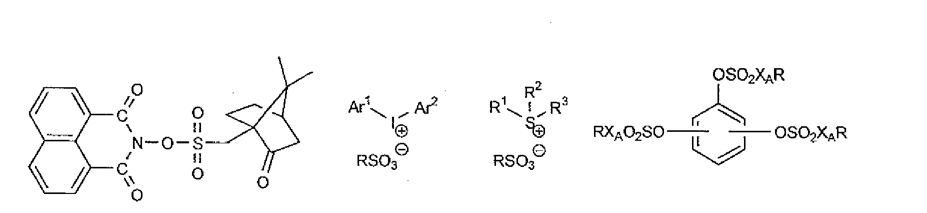 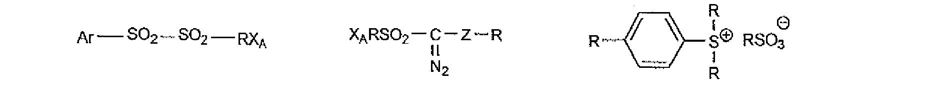 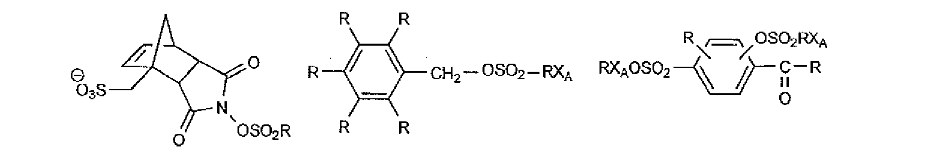 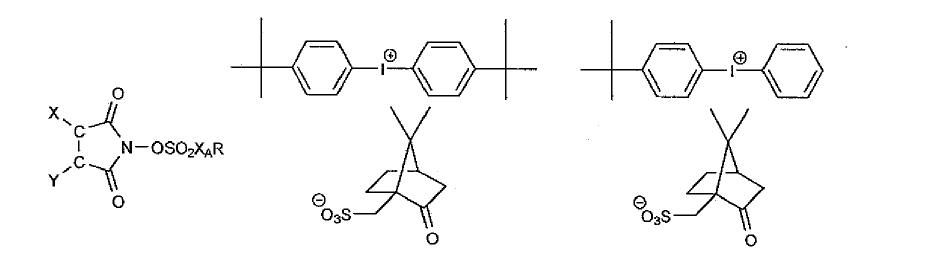 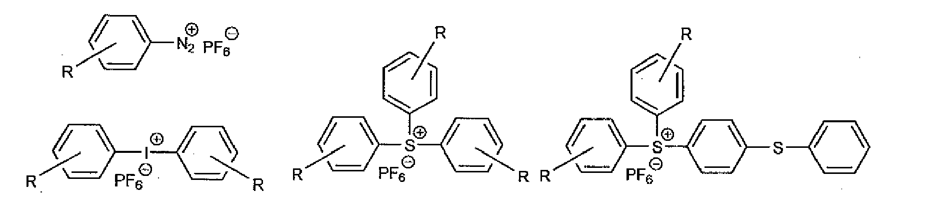 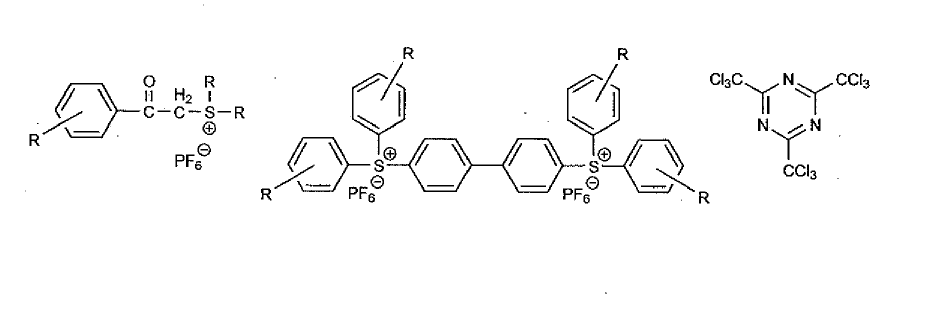 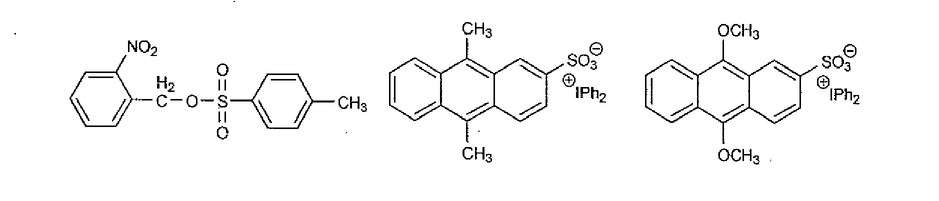 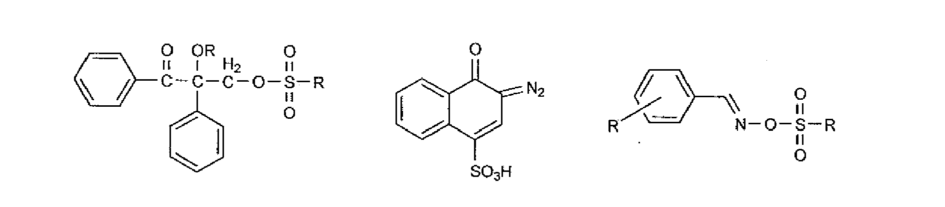 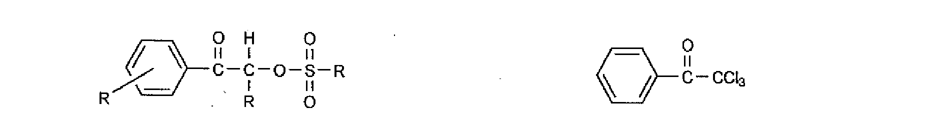 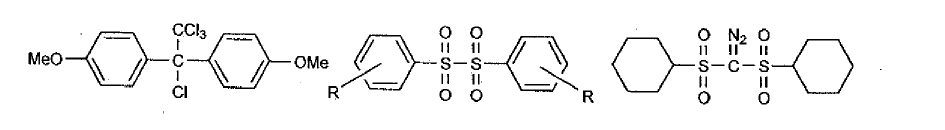 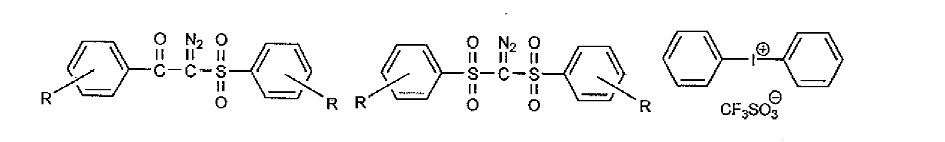 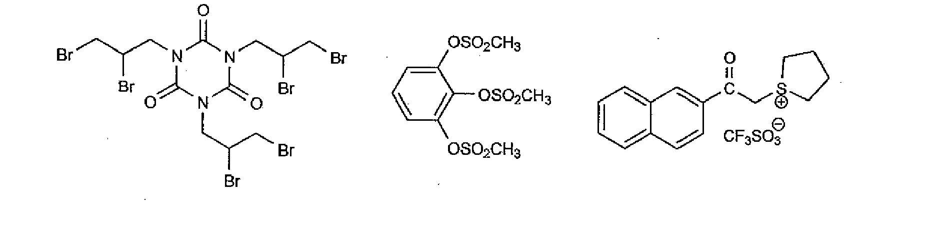 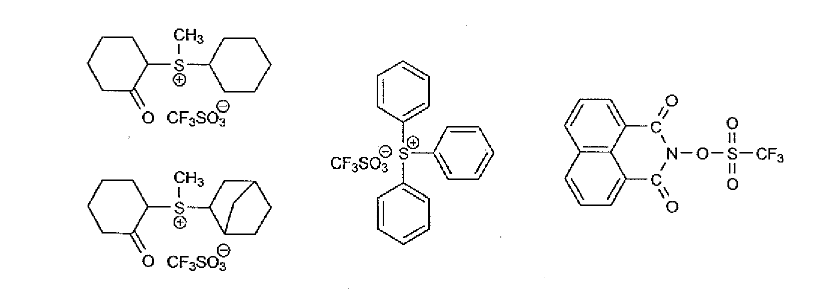 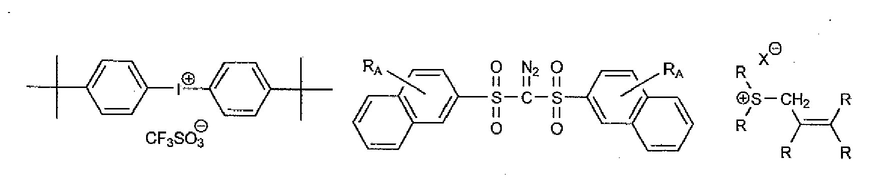 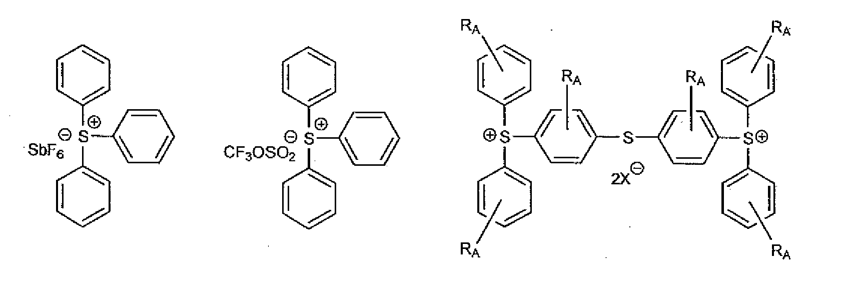 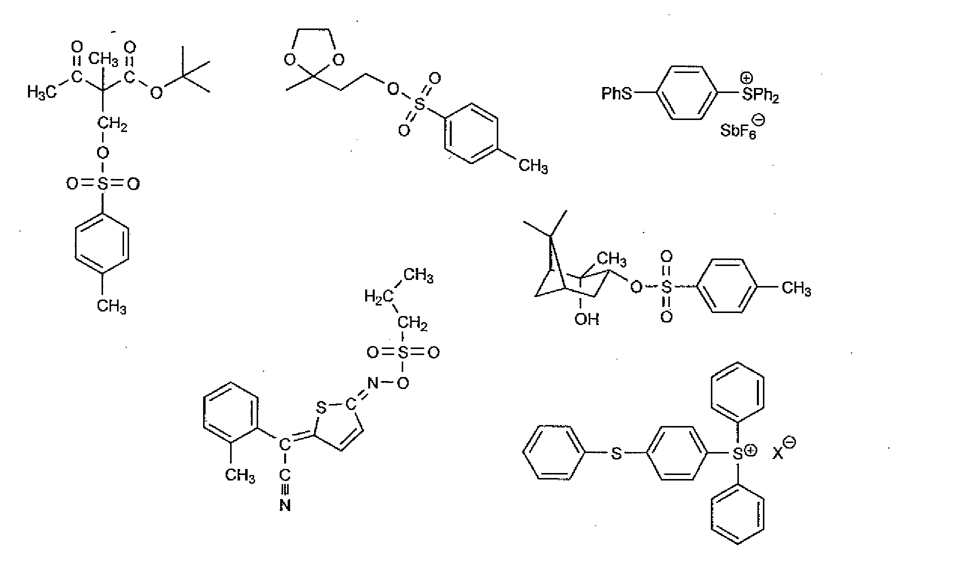 wherein Ar, Ar¹, and Ar² are a substituted or unsubstituted aromatic group having 6 to 20 carbon atoms; R, R¹, R², R³, and R_{A} are a substituted or unsubstituted aromatic group having 6 to 20 carbon atoms, and a substituted or unsubstituted aliphatic group having 1 to 20 carbon atoms; and X, X_{A}, Y, and Z are an aliphatic sulfonium group, a fluorine-containing aliphatic sulfonium group, a tetrafluoroborate group, and a hexafluorophosphonium group.

Usually, a PAG is added in an amount of 0.1 to 20 wt% relative to the amount of a photoresist base material.

As the quenching agent, in addition to known quenching agents, other compounds having the same effect can be generally used. A preferable quenching agent and the amount thereof can be appropriately selected according to the type of a calixresorcinarane compound as a base material, the type of a PAG, and a desired shape and size of minute patterns, and the like.
In the invention, it is preferable to use a basic organic compound as a quenching agent from the viewpoint of solubility in a photoresist composition and dispersibility and stability in a photoresist layer.
As specific examples of the basic organic compound, in addition to pyridines such as quinoline, indole, pyridine, and bipyridine, pyrimidines, pyrazines, piperidine, piperazine, pyrrolidine, 1,4-diazabicyclo[2.2.2]octane, aliphatic amines such as triethylamine and trioctylamine, tetrabutylammonium hydroxide, and the like can be given.

The amount of the quenching agent is usually from 10 to 1 × 10⁻³ wt% of a photoresist base material or from 50 to 0.01 wt% of a PAG.

One example of a method for microfabrication using the photoresist composition of the invention will be described below.
The photoresist composition of the invention is applied to a substrate as a liquid coating composition by spin coating, dip coating, painting, and the like. In a common practice after the application, the coated material is dried by heating at 80°C to 160°C, for example, in order to remove the solvent until the photoresist coating layer becomes non-sticky. To improve adhesion with the substrate, hexamethyldisilazane (HMDS) or the like can be used as an intermediate layer. The conditions can be appropriately adjusted according to the types of a base material and solvent used, a desired film thickness of a photoresist layer, and the like.

After drying, the substrate on which the photoresist coating layer is no longer sticky is exposed through a photomask to EUV or is irradiated with an electron beam by any method to remove protective groups contained in a base material, thereby producing solubility differences between the exposed areas and unexposed areas in the photoresist coating layer. After the exposure, the substrate is baked to increase the solubility differences, followed by development with an alkaline developer in order to form relief images. Patterns processed by ultra-microfabrication can be thus formed on the substrate in this manner. The above conditions can be determined according to the type of the base material or solvent used or a desired thickness of a photoresist layer, or the like.

If the photoresist base material and the composition thereof of the invention are used, patterns with isolated lines of 100 nm or less, particularly 50 nm or less, a 1:1 line-and-space (L/S), holes, etc, can be formed at a high sensitivity, high contrast, and low line edge roughness by ultra-microfabrication using extreme ultraviolet radiation or an electron beam.

### EXAMPLES

### Synthesis Example 1

### [Synthesis of calixresorcinarene compound]

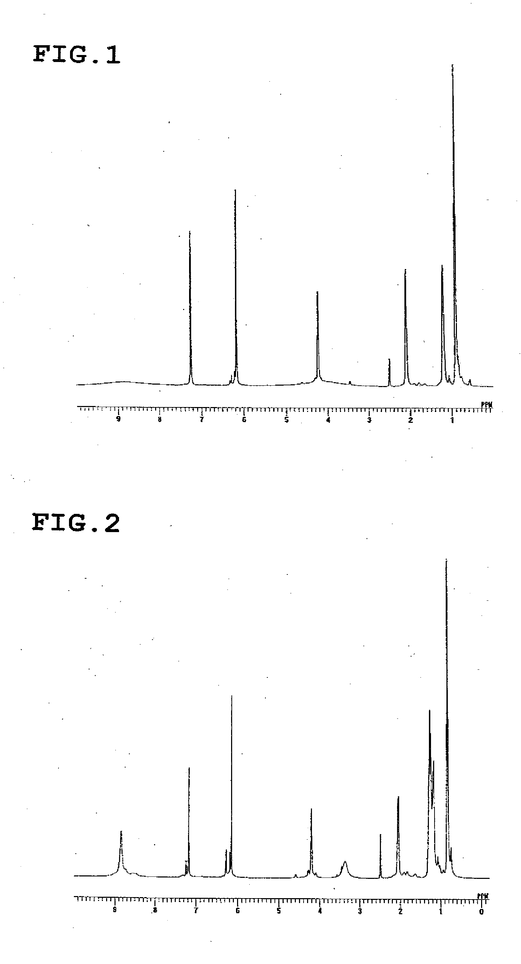

A three-neck flask (volume: 500 ml) equipped with a dripping funnel, a Dimroth condenser, and a thermometer, sufficiently dried and replaced with nitrogen gas, was charged with resorcinol (33 g, 300 mmol) and n-butylaldehyde (21.6 g, 300 mmol) in a nitrogen stream and sealed. Then, distilled methanol (300 ml) was added under a slight pressure of nitrogen gas to obtain a methanol solution. The methanol solution was heated to 75°C on an oil bath while stirring. 18 ml of a concentrated hydrochloric acid solution was slowly added by dripping from the dripping funnel, followed by continued stirring with heating at 75°C for two hours. After completion of the reaction, the mixture was allowed to cool to room temperature, followed by cooling on an ice water bath. The reaction mixture was allowed to stand for one hour. White raw crystals of the target compound were produced and collected by filtration. The crude crystals were washed twice with purified water (100 ml), purified by recrystallization from a mixed solution of ethanol and water, and dried under reduced pressure to obtain a calixresorcinarene compound represented by the following formula (41.8 g, yield: 85%), The structure of this compound was identified by ¹H-NMR (FIG. 1), IR, elementary analysis, and the like.

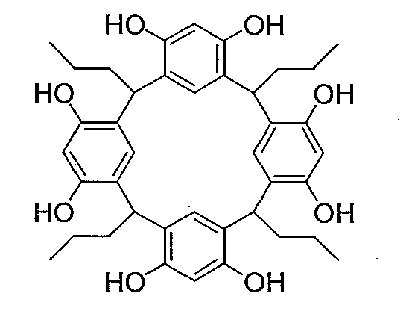

### Synthesis Example 2

### [Synthesis of calixresorcinarene compound]

The calixresorcinarene compound represented by the following formula was synthesized in the same manner as in Synthesis Example 1, except that n-hexylaldehyde was used instead of n-butylaldehyde (yield: 87%). The structure of this compound was identified by ¹H-NMR (FIG. 2).

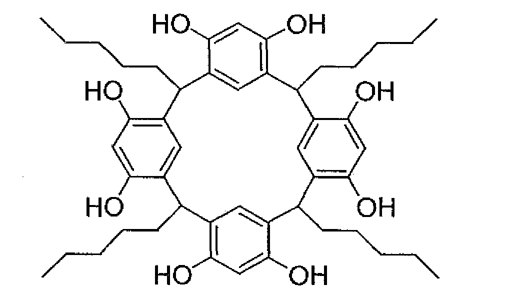

### Synthesis Example 3

### [Synthesis of calixresorcinarene compound]

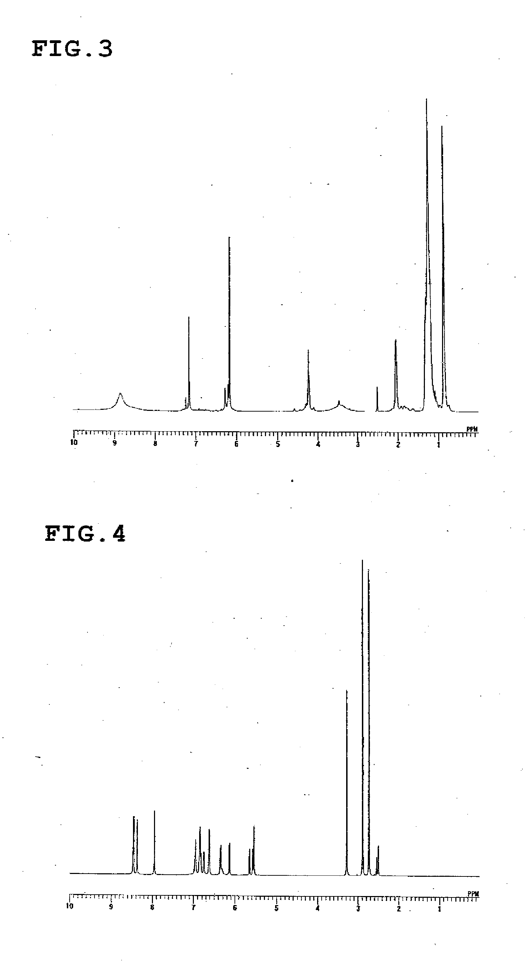

The calixresorcinarene compound represented by the following formula was synthesized in the same manner as in Synthesis Example 1, except that n-octylaldehyde was used instead of n-butylaldehyde (yield: 86%). The structure of this compound was identified by ¹H-NMR (FIG. 3).

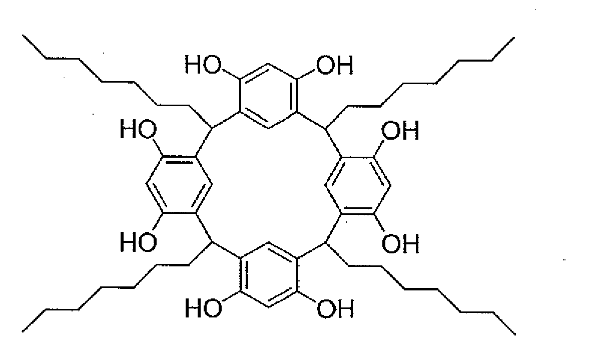

### Synthesis Example 4

### [Synthesis of calixresorcinarene compound]

The calixresorcinarene compound represented by the following formula was synthesized in the same manner as in Synthesis Example 1, except that benzaldehyde was used instead of n-butylaldehyde (yield: 82%). The structure of this compound was identified by ¹H-NMR (FIG. 4).

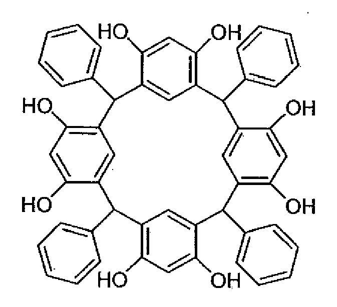

### Synthesis Example 5

### [Synthesis of calixresorcinarene compound]

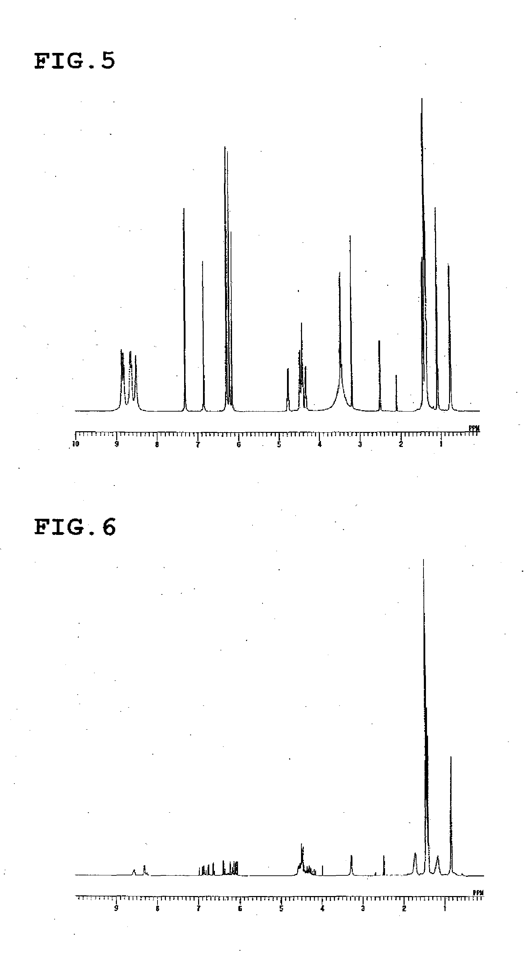

The calixresorcinarene compound represented by the following formula was synthesized in the same manner as in Synthesis Example 1, except that acetoaldehyde was used instead of n-butylaldehyde (yield: 40.2%). The structure of this compound was identified by ¹H-NMR (FIG. 5).

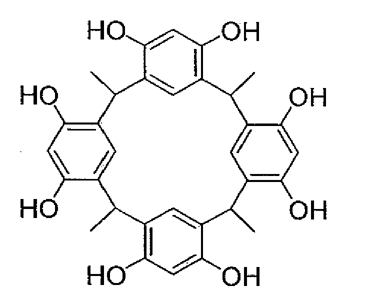

### Example 1

### [Preparation of photoresist base material]

A two-neck flask (volume: 100 ml) equipped with a Dimroth condenser and a thermometer, sufficiently dried and replaced with nitrogen gas, was charged with the calixresorcinarene compound (2.50 g, 3.8 mmol) prepared in Synthesis Example 1, potassium carbonate (3.18 g, 30 mmol), and 15-crown-5 (0.77 g, 3.5 mmol), and sealed. The flask was replaced with nitrogen gas. Next, after adding 38 ml of acetone to prepare a solution, tert-butyl bromoacetate (6.34 g, 4.8 mmol) was added and the mixture was heated to reflux in a nitrogen atmosphere in an oil bath at 75°C while stirring for 24 hours. Thereafter, the mixture was allowed to cool to room temperature and ice water was added, followed by stirring for one hour to obtain a white precipitate. The precipitate was collected by filtration and dissolved in diethyl ether (10 ml). The resulting solution was poured to an aqueous acetic acid solution (0.5 mol/l, 300 ml) to obtain white crystals. The white crystals were collected by filtration and dried under reduced pressure for purification, thereby to obtain a calixresorcinarene compound represented by the following formula (1.5 g, yield: 35%). The resulting compound was used as a photoresist base material.
The structure of this calixresorcinarene compound was identified by ¹H-NMR (FIG. 6), IR, elementary analysis, and the like.

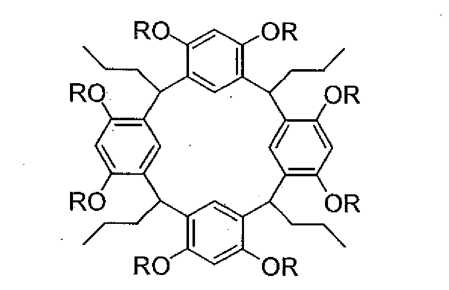

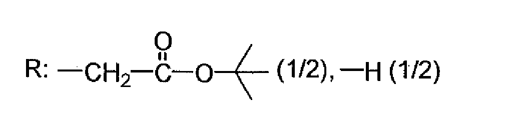

### Example 2

### [Preparation of photoresist base material]

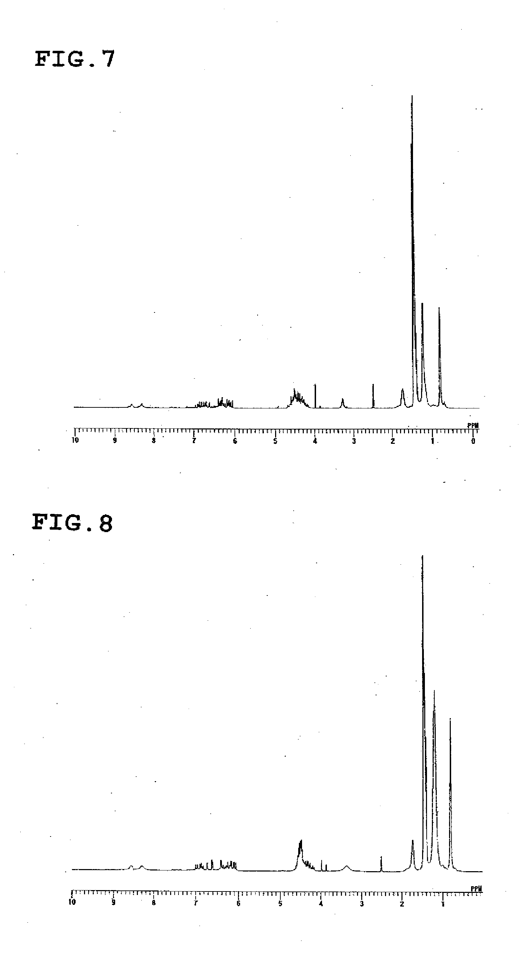

The photoresist base material represented by the following formula was prepared in the same manner as in Example 1, except that the calixresorcinarene compound synthesized in Synthesis Example 2 was used instead of the calixresorcinarene compound synthesized in Synthesis Example 1 (yield: 33%). The structure of this calixresorcinarene compound was identified by ¹H-NMR (FIG. 7).

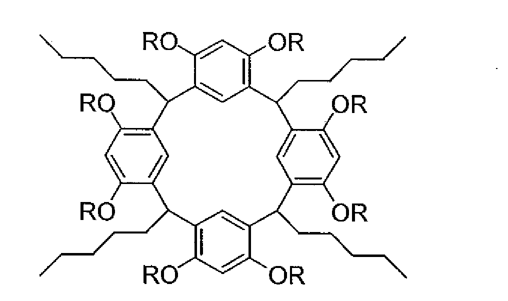

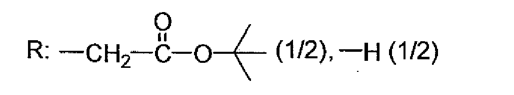

### Example 3

### [Preparation of photoresist base material]

The photoresist base material represented by the following formula was prepared in the same manner as in Example 1, except that the calixresorcinarene compound synthesized in Synthesis Example 3 was used instead of the calixresorcinarene compound synthesized in Synthesis Example 1 (yield: 63%). The structure of this calixresorcinarene compound was identified by ¹H-NMR (FIG. 8).

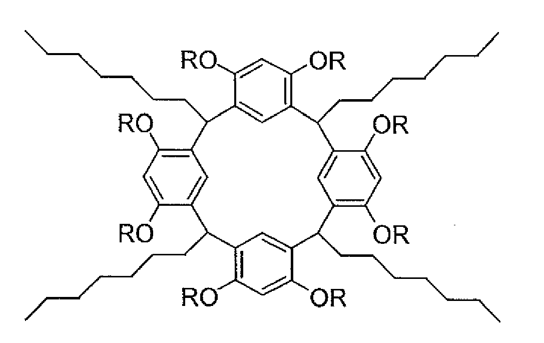

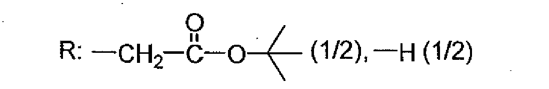

### Example 4

### [Preparation of photoresist base material]

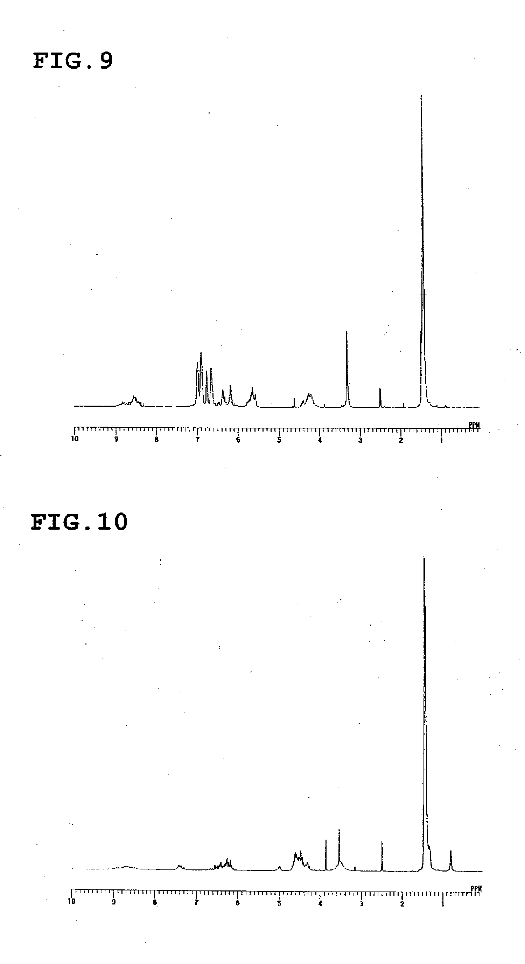

The photoresist base material represented by the following formula was prepared in the same manner as in Example 1, except that the calixresorcinarene compound synthesized in Synthesis Example 4 was used instead of the calixresorcinarene compound synthesized in Synthesis Example 1 (yield: 52%). The structure of this calixresorcinarene compound was identified by ¹H-NMR (FIG. 9).

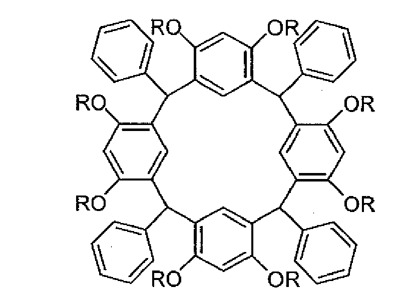

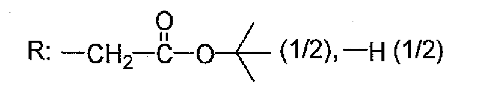

### Examples 5 to 8

### [Preparation of photoresist composition]

A solid mixture consisting of 87 parts by weight of the calixresorcinarene compounds prepared in Examples 1 to 4, 10 parts by weight of triphenylsulfonium trifluoromethanesulfonate, as a PAG, and 3 parts by weight of 1,4-diazabicyclo[2.2.2]octane, as a quenching agent, was dissolved in propylene glycol methyl ether acetate to a solid concentration of 10 wt%, thereby obtaining a photoresist solution.
The photoresist solution was applied onto a silicon wafer by spin coating and baked at 105°C for 180 seconds to form a thin film. The substrate with the coating was exposed to EUV using EUV exposure equipment. Thereafter, the substrate was baked at 100°C for 60 seconds, treated with a 2.38 wt% aqueous tetramethylammonium hydroxide solution for 60 seconds, and washed with purified water, followed by drying with a nitrogen gas stream.
The EUV exposure evaluation was conducted at a BL3 beamline, New SUBARU radiation facility of Laboratory of Advanced Science Technology for Industry, University of Hyogo. Details of the light source and the exposure equipment are described, for example, in T. Watanabe, H. Kinoshita, K. Hamamoto, H. Hada and H. Komano, J. photopolym. Sci. technol., Vol. 17, No. 3, 2004.
The regist outgas was measured by analyzing gas components generated during EUV irradiation by a quadrupol mass spectrometer. The sensitivity was calculated from the relationship between the thickness of a film remained in the irradiated area and the dose of EUV. The irradiated surface roughness was determined with a scanning interatomic force microscope by measuring the surface roughness of a part where a remaining film was present in the irradiated area.
Etching resistance was measured as follows. The photoresist base materials in Examples 1 to 4 were formed into a 500 nm-thick film on a silicon wafer. The film was etched under the conditions of 60 sccm and 300 W by a RIE etching device using CF₄ as an etching gas. Etching rate was calculated using polyhydroxystyrene which contains a tert-butyloxycaxbonylmethyl group (Comparative Example 2) as a reference.
The results are shown in Table 1.

### Comparative Example 1

### [Preparation of photoresist base material]

The photoresist base material represented by the following formula was prepared in the same manner as in Example 1, except that the calixresorcinarene compound synthesized in Synthesis Example 5 was used instead of the calixresorcinarene compound synthesized in Synthesis Example 1 (yield: 68%). The structure of this calixresorcinarene compound was identified by ¹H-NMR (FIG. 10).

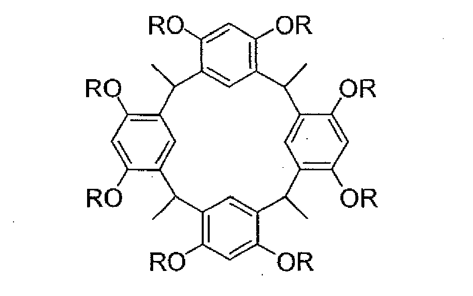

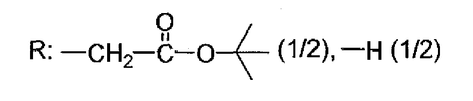

### Comparative Example 2

### [Preparation of photoresist base material]

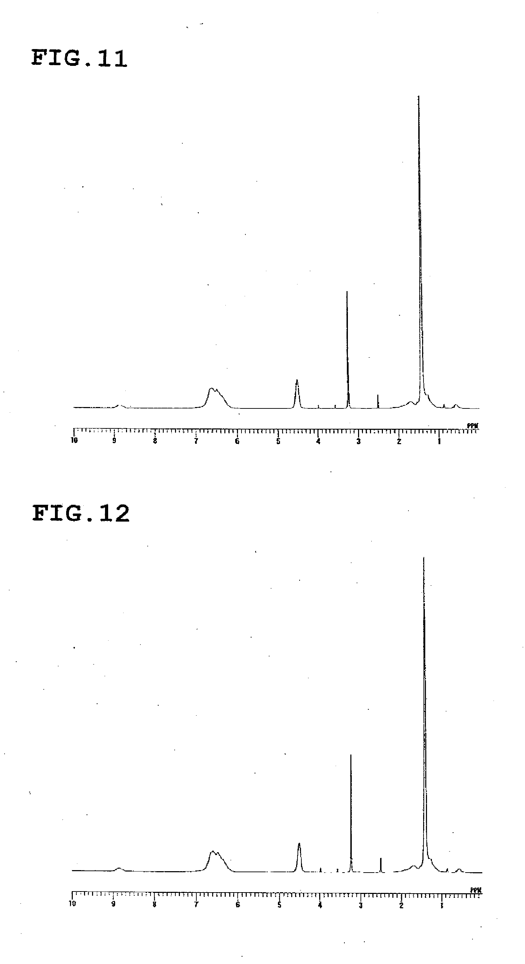

Polyhydroxystyrene represented by the following formula comprising 60% of tert-butyloxycarbonylmethyl and 40% of hydrogen was prepared in the same manner as in Example 1, except that polyhydroxystyrene with a molecular weight of 8,000 (the ¹H-NMR chart of which is shown in FIG. 11) was used instead of the calixresorcinarene compound synthesized in Synthesis Example 1. The structure of this compound was identified by ¹H-NMR (FIG. 12).

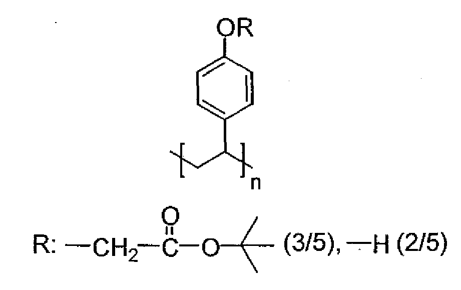

### Comparative Examples 3 and 4

### [Preparation of photoresist composition]

A photoresist solution was prepared from the photoresist base material prepared in Comparative Examples 1 and 2 in the same manner as in Example 5. A thin film was formed from the solution on a silicon wafer.
The resulting thin film was evaluated for the regist outgas, sensitivity, roughness of the irradiated surface, and etching rate in the same manner as in Example 5. The results obtained are shown in Table 1.

**Table 1**

| Photoresist composition | Example 5 | Example 6 | Example 7 | Example 8 | Comparative Example 3 | Comparative Example 4 |
|---|---|---|---|---|---|---|
| Photoresist base material | Example 1 | Example 2 | Example 3 | Example 4 | Comparative Example 1 | Comparative Example 2 |
| Regist outgas (Pa) | 6.3 × 10⁻⁵ | 1.2 × 10⁻⁴ | 2.4 × 10⁻⁴ | 7.1 × 10⁻⁵ | 1.2 × 10⁻⁵ | 5.2 × 10⁻⁴ |
| Sensitivity (mJ/cm²) | 19 | 24 | 45 | 1.5 | 11 | 2 |
| Roughness of irradiated surface (nm-Rms) | 2.1 | 2.3 | 2.6 | 2.4 | 2 | 7.7 |
| Etching resistance | Good | Good | Good | Very good | Poor | - |

As for the evaluation of etching resistance in the table, an etching rate of 0.95 or less was indicated as very good, an etching rate of 1.05 to 0.95 was indicated as good, and an etching rate of 1.05 or more was indicated as poor.

### INDUSTRIAL APPLICABILITY

The photoresist base material and the composition thereof of the invention can be suitably used in the fields of electricity and the electronics such as a semiconductor, the optical field, and the like. This photoresist base material and the composition thereof can remarkably improve the performance of semiconductor devices such as an ULSI.

## Claims

1. A calixresorcinarene compound represented by the following formula (1): 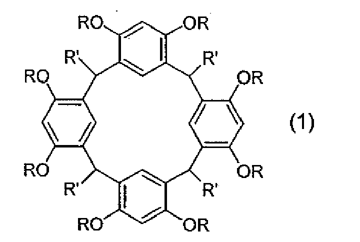 wherein eight Rs are n (n is an integer of 1 to 7) substituents that are one or more types of substituents selected from groups represented by the following formula (2), and m (m is an integer shown by 8-n) hydrogen atoms; and R's, which may be the same or different, are each a straight-chain aliphatic hydrocarbon group having 2 to 12 carbon atoms, a branched aliphatic hydrocarbon group having 3 to 12 carbon atoms, a phenyl group, a p-phenylphenyl group, a p-tert-butylphenyl group, and an aromatic group represented by the following formula (3), or a substituent formed by combining two or more of these substituents: 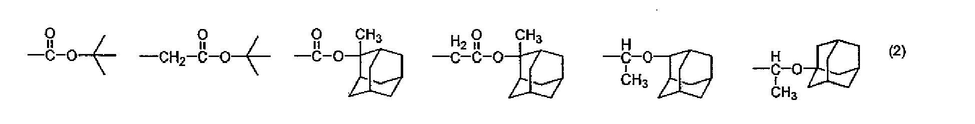 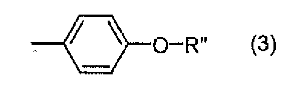 wherein R" is a hydrogen atom or a substituent selected from the substituents represented by the formula (2).

2. The calixresorcinarene compound according to claim 1, wherein R' is ethyl, n-propyl, iso-propyl, n-butyl, iso-butyl, tert-butyl, n-pentyl, n-hexyl, n-heptyl, n-octyl, n-nonyl, n-decyl, n-undecyl or n-dodecyl.

3. The calixresorcinarene compound according to claim 1 wherein R' is phenyl, p-phenylphenyl or p-tert-butylphenyl and a calixresorcinarene compound represented by the following formula (4): 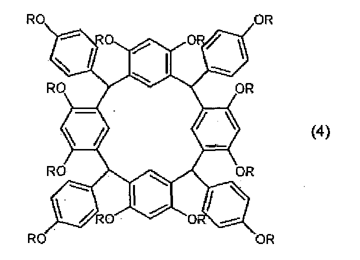 wherein twelve Rs are n' (n' is an integer of 1 to 11) substituents that are one or more types of substituents selected from groups represented by the following formula (2), and m' (m' is an integer represented by 12-n') hydrogen atoms.

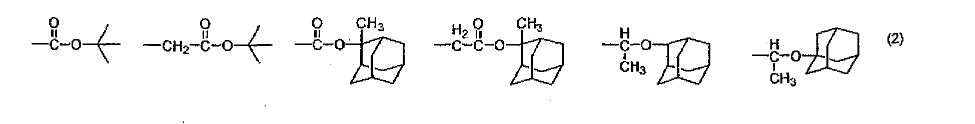

4. A photoresist base material comprising the calixresorcinarene compound according to any one of claims 1 to 3.

5. A photoresist composition comprising the photoresist base material according to claim 4 and a solvent.

6. A microfabrication method by lithography which uses the photoresist composition according to claim 5.

7. A microfabrication method by extreme ultraviolet lithography which uses the photoresist composition according to claim 5.

8. A semiconductor device which is formed by the microfabrication method according to claim 6 or 7.
